# EUROPEAN PATENT SPECIFICATION

(11) **EP 2 088 986 B1**
(45) Date of publication and mention of the grant of the patent: **13.04.2011**
(21) Application number: 07832970.3
(22) Date of filing: 28.11.2007
(51) Int. Cl.: A61K 8/06, A61K 8/26, A61K 8/41, A61K 8/44, A61K 8/63, A61K 8/68

(54) **SKIN EXTERNAL PREPARATION IN THE FORM OF WATER-IN-OIL EMULSION COMPRISING CERAMIDE**
TOPISCHES HAUTPRÄPARAT IN FORM EINER CERAMID ENTHALTENDEN WASSER-IN-ÖL-EMULSION
PREPARATION CUTANEE A USAGE EXTERNE SOUS FORME D'UNE EMULSION EAU DANS HUILE COMPRENANT UN CERAMIDE

(30) Priority: 14.12.2006 JP 2006337003; 14.12.2006 JP 2006337020; 14.12.2006 JP 2006337022; 14.12.2006 JP 2006337027
(43) Date of publication of application: 19.08.2009
(73) Proprietor: Pola Chemical Industries Inc., Shizuoka-shi, Shizuoka 422-8009 (JP)
(72) Inventor: AKATSUKA, Hidetaka, Yokohama-shi Kanagawa 244-0812 (JP); SEINO, Ayako, Yokohama-shi Kanagawa 244-0812 (JP); SETO, Tadahito, Yokohama-shi Kanagawa 244-0812 (JP); SUZUKI, Masashi, Yokohama-shi Kanagawa 244-0812 (JP)
(74) Representative: Westwood, Joanna
(86) International application number: PCT/JP2007/073428
(87) International publication number: WO 2008/072507

(56) References cited:
- EP-A- 1 443 108
- US-B1- 6 824 785
- DATABASE WPI Week 199347 Derwent Publications Ltd., London, GB; AN 1993-374518 XP002475220 & JP 05 279238 A (AJINOMOTO KK) 26 October 1993 (1993-10-26)
- DATABASE WPI Week 200627 Derwent Publications Ltd., London, GB; AN 2006-257974 XP002475221 & JP 2006 089420 A (AJINOMOTO TAKARA CORP) 6 April 2006 (2006-04-06)

## Description

### Technical Field

The present invention relates to skin external preparations, more specifically to skin external preparations in the form of water-in-oil emulsion.

### Background Art

At the present day, one of the major problems in the dermatological field is an exponential increase in atopic dermatitis. The causes of atopic dermatitis have not been clarified in detail, but atopic dermatitis is considered to be caused by a plurality of factors such as hypersensitive reaction of the skin to broad chemical substances. Therefore, skin care to prevent introduction of chemical substances into a living body is required. However, a component contained in a skin external preparation to be administered for skin care may provide a stimulus to the skin. In order to prevent the stimulus, it is considered to be effective to enhance the barrier function on the bottom of the horny layer by delivering a ceramide or the like to near the dermis across the horny layer (Patent Documents 1 and 2). However, ceramides have low solubility to water and low solubility to oil and have many difficulties in production of formulations. Accordingly, a technology for promoting solubility to a base of formulation using a pseudo-ceramide (ceramide derivative) has been developed (Patent Document 3). However, even if a skin external preparation produced by such a technology is administered to the skin, it is very difficult to deliver a ceramide to near the dermis.

As means for delivering a ceramide across the horny layer, means for forming a microsphere of a lipid bilayer membrane together with a phospholipid or the like has been developed (Patent Document 4). The technology facilitates delivery of a ceramide across the horny layer, but even applying the technology, it is difficult to sufficiently deliver a ceramide across the horny layer and did not achieve sufficient exertion of the effects of ceramides in a skin external preparation.

On the other hand, organically modified clay minerals such as organically modified bentonite and dimethyldistearylammonium chloride-modified hectorite are used for stabilization of a water-in-oil emulsion (Patent Documents 5 and 6). However, a skin external preparation comprising an organically modified clay mineral together with a ceramide is unknown at all.

Meanwhile, a skin external preparation in the form of a water-in-oil emulsion comprising a diester of an N-acylated acidic amino acid is known (see Patent Documents 7, 8, and 9). However, a skin external preparation in the form of a water-in-oil emulsion comprising a diester of an N-acylated acidic amino acid together with a ceramide is unknown at all.

Phytosterols are lipids which are contained in a plant body and have a sterol skeleton, and representative examples thereof include stigmasterol, sitosterol, and campesterol. Such components are known to have an effect to synchronize metabolic cycles of epidermal cells in skin and an ability to enhance the strength of a septum having a bilayer membrane structure in a liposome or the like (Patent Documents 10 and 11). However, a skin external preparation in the form of a water-in-oil emulsion comprising a phytosterol together with a ceramide is unknown at all.

Meanwhile, cross-linked silicones are generally used for a skin external preparation such as a cosmetic, and a combination use of a cross-linked silicone and a ceramide is known (for example, see Patent Document 12). However, a skin external preparation in the form of a water-in-oil emulsion comprising an organically modified clay mineral and a cross-linked silicone is unknown at all.

[Patent Document 1] WO 99/45900
[Patent Document 2] WO 02/006225
[Patent Document 3] JP 2002-332208 A
[Patent Document 4] JP 2005-522463 A
[Patent Document 5] JP 11-246354 A
[Patent Document 6] WO 2003/041664 A
[Patent Document 7] JP 5-279238 A
[Patent Document 8] JP 11-76799 A
[Patent Document 9] JP 8-20529 A
[Patent Document 10] JP 2004-230173 A
[Patent Document 11] JP 8-40823 A
[Patent Document 12] JP 2001-151628 A

### Disclosure of the Invention

An object of the present invention is to provide a technology for delivering a ceramide across the horny layer to sufficiently exert the effects of ceramide. Specifically, an object of the present invention is to provide a skin external preparation comprising a ceramide, which enables sufficient delivery of a ceramide in the skin external preparation across the horny layer to near the dermis when the skin external preparation is applied on the skin surface.

The inventors of the present invention have been made extensive studies to achieve the above-mentioned objects. As a result, they have found out that a skin external preparation in the form of a water-in-oil emulsion comprising a ceramide, an organically modified clay mineral, a diester of N-acylglutamic acid, and a phytosterol can sufficiently exert the effects of the ceramide, thus accomplished the present invention.

That is, the present invention is as follows:

(1) a skin external preparation in the form of a water-in-oil emulsion, comprising a ceramide, an organically modified clay mineral, a diester of N-acylglutamic acid, and a phytosterol;
(2) the skin external preparation according to Item (1), further comprising a crosslinked dimethicone;
(3) the skin external preparation according to Item (1) or (2), wherein the ceramide is comprised in the form of a liposome or a niosome;
(4) the skin external preparation according to any one of Items (1) to (3), wherein the organically modified clay mineral is hectorite modified with a quaternary ammonium salt;
(5) the skin external preparation according to any one of Items (1) to (4), wherein the diester of N-acylglutamic acid is di(phytosteryl/octyldodecyl) N-lauroylglutamate;
(6) the skin external preparation according to any one of Items (1) to (5), wherein the skin external preparation is a cosmetic for protecting skin from external chemical irritation; and
(7) the skin external preparation according to any one of Items (1) to (5), wherein the skin external preparation is for ameliorating or preventing atopic dermatitis.

### Best Mode for carrying out the Invention

A skin external preparation of the present invention comprises a ceramide, an organically modified clay mineral, a diester of N-acylglutamic acid, and a phytosterol as essential components.

### (1) Ceramide

A skin external preparation of the present invention comprises a ceramide as an essential component. In general, seven types of ceramides, ceramide types 1-7, are known, and all of the ceramides can be used. Of those, ceramide types 2 and 3 are more preferable, and ceramide type 2 is particularly preferable, and N-stearoyl-dihydroxy-sphingosine is especially preferable. Commercialized products of the ceramides are available, and they may be purchased and used. Preferable examples of the products include "Ceramide I" (manufactured by Cosmo Pharm) containing N-(27-octadecanoyloxy-heptacosanoyl)-phytosphingosine (type 1) as a component, "Ceramide TIC-001" (manufactured by Takasago International Corporation) containing N-stearoyl-dihydroxy-sphingosine (type 2) as a component, "Ceramide III" (manufactured by Cosmo Pharm) containing N-stearoyl-phytosphingosine (type 3) as a component, "Ceramide IIIA" (manufactured by Cosmo Pharm) containing N-linoleoyl-phytosphingosine (type 3) as a component, "Ceramide IIIB" (manufactured by Cosmo Pharm) containing N-oleoyl-phytosphingosine (type 3) as a component, and "Ceramide VI" (manufactured by Cosmo Pharm) containing N-2-hydroxystearoyl-phytosphingosine (type 6) as a component.

Those ceramides may be contained singly or in combination of two or more. The ceramide content in a skin external preparation of the present invention is preferably 0.02 to 5% by mass, more preferably 0.01 to 2% by mass.
A ceramide has an effect of reinforcing a bond between horny layer cells to improve skin barrier function.

### (2) Organically modified clay mineral

A skin external preparation of the present invention comprises an organically modified clay mineral as an essential component. The term "organically modified" as used herein refers to allowing an organic compound to strongly or loosely bind to a part of a clay mineral via a covalent bond or an ion bond to impart a part or all of properties of the organic compound to the clay mineral. Examples of a method of organically modifying a clay mineral include: a method comprising preparing a quaternary ammonium salt of an organic compound and a clay mineral and allowing the organic compound to bind to an anion moiety of the clay mineral; and a method comprising preparing a carboxylic acid of an organic compound and a clay mineral and allowing the organic compound to bind to a cation moiety of the clay mineral, and of those, the method comprising preparing a quaternary ammonium salt and a clay mineral and allowing the organic compound to bind to an anion moiety of the clay mineral is particularly preferable.

The quaternary ammonium salt to be used for organically modifying a clay mineral is not particularly limited, but examples thereof include a compound referred to as "quaternium". The quaternium is a low-molecular-weight quaternary ammonium salt. In the present invention, it is preferable to use a compound registered in the International Nomenclature Cosmetic Ingredients (INCI) or a compound contained in a conventional skin external preparation as a quaternium. Among those quaterniums, a quaternary ammonium salt represented by the following chemical formula (1) is preferably used, and particularly preferable examples thereof include stearyltrimethylammonium chloride, dimethyldistearylammonium chloride, and benzyldimethylstearylammonium chloride. In particular, a clay mineral organically modified with stearyltrimethylammonium chloride, dimethyldistearylammonium chloride, etc. is preferable because it can form a stable water-in-oil emulsion.

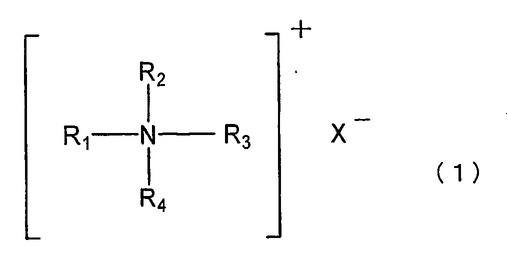

In the Chemical formula (1), R₁ represents an alkyl group having 10 to 22 carbon atoms, R₂ represents an alkyl group having 10 to 20 carbon atoms, methyl group, or benzyl group, R3 and R4 represent methyl groups, and X represents a halogen atom.

On the other hand, the clay mineral to be organically modified with a quaternary ammonium salt (unmodified clay mineral) is not particularly limited as long as it is a clay mineral used for a conventional skin external preparation. Examples of the clay mineral used for a conventional skin external preparation include: smectite including hectorite, bentonite, and montmorillonite; kaolinite; illite; marine clay mineral (sea clay); desert rose clay mineral; and pascalite. Of those, hectorite, bentonite, montmorillonite, and kaolinite are preferable because of the excellent ability to stabilize a water-in-oil emulsion.

As a method of producing an organically modified clay mineral, a method of modifying a clay mineral with a quaternary ammonium salt will be described as an embodiment. An unmodified clay mineral is dispersed in a disperse medium. The disperse medium is preferably a water-based solvent and may be water. A quaternary ammonium salt is further added to the disperse medium containing the unmodified clay mineral, followed by mixing well. The quaternary ammonium salt may be added after dissolved in water. The amount of quaternary ammonium salt is preferably 0.1 to 20% by mass, more preferably 0.5 to 15% by mass of the amount of dispersed unmodified clay mineral. A skin external preparation having such a composition can provide excellent feeling to skin. After stirring, the dispersoids are removed by filtration, followed by dewatering and drying, to thereby yield an organically modified clay mineral to be used in the present invention. Alternatively, the organically modified clay mineral to be used in the present invention can be obtained by performing vacuum concentration to remove the disperse medium without removing the dispersoids by filtration, followed by drying. The resultant organically modified clay mineral is pulverized into a desired size. The particle diameter is preferably 1 to 1,000 µm.

The organically modified clay mineral to be used in the present invention may be prepared as described above or may be a commercially available product. Some of commercially available organically modified clay minerals are used for a skin external preparation such as a cosmetic. Preferable examples of the commercially available organically modified clay minerals include dimethyldistearylammonium-modified hectorite sold under the name of "Bentone 38V" from Elementis and benzyldimethylstearylammonium-modified hectorite sold under the name of "Bentone 27" from Elementis.

The content of organically modified clay mineral in the skin external preparation of the present invention is preferably 0.5 to 10% by mass, more preferably 1 to 5% by mass. Meanwhile, the content of organically modified clay mineralis preferably 0.01 to 100 times by mass, more preferably 0.05 to 50 times by mass that of ceramide.
The organically modified clay mineral works to form water-in-oil emulsion having a high internal phase.

### (3) Diester of N-acylated gluatamic acid

A skin external preparation of the present invention comprises a diester of N-acylated gluatamic acid as an essential component. The number of carbon atoms of the acyl group in the diester of N-acylated glutamic acid is preferably 10 to 30, and more preferably 12 to 18. In addition, the acyl group may be saturated or unsaturated group. For example, 2-ethylhexanoyl group, lauroyl group, myristoyl group, palmitoyl group, stearoyl group, behenoyl group, oleoyl group, isostearoyl group, linolenoyl group are preferable. Of those, lauroyl group is particularly preferable.

Further, the diester of N-acylated glutamic acid is preferably a dialkyl ester of N-acylated glutamic acid. The number of carbon atoms of each alkyl group forming the dialkyl ester is preferably 1 to 30, and more preferably 12 to 18. The alkyl groups in one molecule of diester may be the same or different. The alkyl group may form branched, linear, or ring structure. For example, octyl group, lauryl group, cetyl group, stearyl group, isostearyl group, behenyl group, octyldodecyl group, phytosteryl groups such as campesteryl group and sitosteryl group, and cholesteryl group are preferable.

A skin external preparation of the present invention may comprise one kind or in combination of two or more kinds of dieters of N-acylated glutamic acid. For example, di(cholesteryl/behenyl/octyldodecyl) N-lauroyl-L-glutamate, di(choresteryl/octyldodecyl) N-lauroyl-L-glutamate, di(phitosteryl/behenyl/octyldodecyl) N-lauroyl-L-glutamate, di(phitosteryl/octyldodecyl) N-lauroyl-L-glutamate are preferable. Di(cholesteryl/octyldodecyl) N-lauroyl-L-glutaimate is a particularly preferable.

A diester of N-acylated glutamic acid can be produced by condensing glutamic acid and an acyl chloride in the presence of an alkaline to produce N-acylglutamic acid and performing dehydration condensation between N-acylglutamic acid and an alcohol corresponding to a group that forms an ester together with N-acylglutamic acid in the presence of a base or an acid and, if necessary, in the presence of a solvent. A diester of N-acylated glutamic acid may be synthesized as described above and may be commercially available as a cosmetic material, and such a commercially available product may be purchased and used. Examples of a particularly preferable commercially available product include "Eldew PS-203"(di(phytosteryl/octyldecyl) N-lauroyl-L-glutamate), "Eldew CL-301" (di(cholesteryl/behenyl/octyldodecyl) N-lauroylglutamate), "Eldew C1-202" (di(cholesteryl/octyldodecyl) N-lauroylglutamate), and "Eldew PS-304" (di(phytosteryl/behenyl/octyldodecyl) N-lauroylglutamate) sold by Ajinomoto Co., Inc., and of those, "Eldew PS-203" is particularly preferable.

The content of diester of N-acylated glutamic acid in a skin external preparation of the present invention is preferably 0.005 to 10% by mass, more preferably 0.01 to 5% by mass. Meanwhile, the content of diester of N-acylated glutamic acid is preferably 0.01 to 100 times by mass, preferably 0.1 to 70 times by mass that of ceramide.
The diester of N-acylated glutamic acid exerts the effect of significantly improving delivery of a ceramide comprised in the skin external preparation to near the dermis.

### (4) Phytosterol

A skin external preparation of the present invention comprises a phytosterol as an essential component. The phytosterol is a compound having a sterol skeleton derived from a plant, and examples thereof include β-sitosterol, stigmasterol, and campesterol. A mixture of those compounds can be obtained by extraction and purification from a plant. Commercialized products of such a mixture are available, and the products may be purchased and used. Preferable examples of the products include "Phytosterol S" (a mixture of β-sitosterol, stigmasterol, and campesterol) sold by Tama Biochemical Co., Ltd.

Those components may be comprised singly or in combination of two or more. Meanwhile, β-sitosterol, stigmasterol, and campesterol are preferably used in combination. The content of phytosterol in the skin external preparation of the present invention is preferably 0.01 to 1% by mass, more preferably 0.05 to 0.2% by mass. Meanwhile, the content of phytosterol in a skin external preparation of the present invention is preferably 0.5 to 5 times by mass, more preferably 1 to 2 times by mass that of ceramide.
Such a component works to stabilize a ceramide in a skin external preparation of the present invention.

### (5) Crosslinked dimethicone

A skin external preparation of the present invention preferably further comprises a crosslinked dimethicone as an essential component. The crosslinked dimethicone has crosslinked structure on polymerization chains of dimethylpolysiloxane, which are linking to another polymerization chains. A crosslinked dimethicone can be produced by mixing methyltrialkoxysilane in dimethylalkoxysilane in production of dimethylpolysiloxane.

Commercialized products of the crosslinked dimethicone are available, and the products may be purchased and used. Examples of such products include "Silicone KSG-16" manufactured by Shin-Etsu Chemical Co., Ltd. (a cyclomethicone solution containing 25% of crosslinked dimethicone), and such a product may be purchased and used.

The content of crosslinked dimethicone in a skin external preparation of the present invention is preferably 0.1 to 10% by mass, more preferably 0.5 to 5% by mass. Meanwhile, the content of crosslinked dimethicone is preferably 0.01 to 50 times by mass, more preferably 0.1 to 30 times by mass that of ceramide.
The crosslinked dimethicone exerts the effect of improving storage stability of a skin external preparation.

### (6) Skin external preparation of the present invention

A skin external preparation of the present invention may comprise a ceramide in a generally known form, preferably in the form of a liposome or a niosome, particularly preferably in the form of a liposome.

Both of the liposome and niosome are microspheres having a hollow construction and septum of a lipid bilayer membrane construction. The term "liposome" refers to a microsphere comprising a phospholipid as a component of the lipid bilayer membrane, and the term "niosome" refers to a microsphere comprising no phospholipid as a component of the lipid bilayer membrane.

A ceramide comprised in a skin external preparation in the form as described above can be easily delivered across the horny layer when the skin external preparation is applied to the skin surface. The phospholipid comprised in a liposome is not particularly limited as long as it can be used in conventional skin external preparations, and preferable examples thereof include lecithin, phosphatidic acid, phosphatidylethanolamine, phosphatidylinositol, phosphatidylserine, phosphatidylglycerol, which are originated from an egg-yolk or a soy bean and lisoforms, hydrogenated products, and hydroxides thereof.
The content of phospholipid in a liposome is preferably 0.5 to 10 times by mass, more preferably 1 to 3 times by mass that of ceramide. Meanwhile, the content of phospholipid is preferably 0.01 to 5% by mass, more preferably 0.1 to 2% by mass in a skin external preparation.

In addition, a liposome preferably contains a polyol as a component. Examples of the polyol include 1,3-butanediol and glycerine.

Moreover, the content of polyol in the liposome is preferably 0.05 to 70 times by mass, more preferably 0.1 to 50 times by mass that of ceramide. Meanwhile, the amount of polyol comprised as a component of the liposome is preferably 0.5 to 10% by mass, more preferably 1 to 5% by mass of the amount of a skin external preparation.

Moreover, a liposome preferably comprises a sterol other than the above-mentioned phytosterols as a component. For example, a liposome preferably comprises cholesterol or the like, and the content is 0.5 to 5 times by mass, more preferably 1 to 2 times by mass that of ceramide.

A liposome and a niosome can be produced by melting the above-mentioned components, decanting the melt to an aqueous carrier with stirring to prepare a crude liposome or a crude niosome, and granulating the product using an extruder, a microfluidizer, etc.

A liposome or a niosome may be dispersed in the outer oil phase or in the inner aqueous phase of a skin external preparation of the present invention, and it is particularly preferably dispersed in the inner aqueous phase.

A skin external preparation of the present invention comprises an organically modified clay mineral in the outer oil phase in the form of a water-in-oil emulsion

A skin external preparation of the present invention may comprise diester of N-acylated glutamic acid in the outer oil phase in the form of a water-in-oil emulsion.
In addition, in the case where a skin external preparation of the present invention comprises the ceramide in the form of a liposome or a niosome, diester of N-acylated glutamic acid may be comprised in the membrane of the liposome or the niosome, or in the outer oil phase in the form of a water-in-oil emulsion, or in both of them. diester of N-acylated glutamic acid is preferably comprised in both the membrane of a liposome or a niosome and the outer oil phase in the form of a water-in-oil emulsion.

A skin external preparation of the present invention may comprise a phytosterol in the outer oil phase in the form of a water-in-oil emulsion.
Moreover, in the case where a skin external preparation of the present invention comprises the ceramide in the form of a liposome or a niosome, a phytosterol is preferably comprised in the membrane of a liposome or a niosome.

A skin external preparation of the present invention, a crosslinked dimethicone is comprised in the outer oil phase in the form of a water-in-oil emulsion.

A skin external preparation of the present invention may comprise a ceramide in the inner aqueous phase in the form of a water-in-oil emulsion at high stability. When a skin external preparation of the present invention is applied to the skin surface, an oil film is formed on the skin surface, and the blocking property of the oil film contributes to delivery of a ceramide across the horny layer.

The form of a skin external preparation of the present invention is not particularly limited as long as the preparation can be administered to skin externally, and preferable examples thereof include cosmetics including quasidrugs, skin external drugs, and skin external articles. Of those, the cosmetics or skin external drug are particularly preferable.
Meanwhile, a skin external preparation of the present invention is particularly preferably for protecting the skin from external chemical irritation and ameliorating or preventing atopic dermatitis. The term "preventing" as used herein refers to both prevention of occurrence of symptoms and prevention of worsening of symptoms.

A skin external preparation of the present invention may comprise an arbitrary component generally used in skin external preparations other than the above components. Examples of the arbitrary components include oils or waxes such as macadamia nut oil, avocado oil, corn oil, olive oil, rapeseed oil, sesame oil, castor oil, safflower oil, cottonseed oil, jojoba oil, coconut oil, palm oil, liquid lanolin, hydrogenated coconut oil, hydrogenated oil, haze wax, hydrogenated castor oil, beeswax, candelilla wax, carnauba wax, ibota wax, lanolin, reduced lanolin, hard lanolin, jojoba wax, dimethicone, and polyoxyethylene-modified dimethylpolysiloxane; hydrocarbons such as a liquid paraffin, squalan, pristane, ozokerite, paraffin, ceresin, vaseline, and microcrystalline wax; higher fatty acids such as oleic acid, isostearic acid, lauric acid, myristic acid, palmitic acid, stearic acid, behenic acid, and undecylenic acid; higher alcohols such as cetyl alcohol, stearyl alcohol, isostearyl alcohol, behenyl alcohol, octyldodecanol, myristyl alcohol, and cetostearyl alcohol; synthetic ester oils such as cetyl isoctanoate, triglyceride isoctanoate, isopropyl myristate, hexyldecyl isostearate, diisopropyl adipate, di-2-ethylhexyl sebacate, cetyl lactate, diisostearyl malate, ethylene glycol di-2-ethyl hexanoate, neopentylglycol dicaprate, glyceryl di-2-heptyl undecanoate, glyceryl tri-2-ethylhexanoate, trimethylolpropane tri-2-ethylhexanoate, trimethylolpropane triisostearate, and pentane erythritol tetra-2-ethylhexanoate; silicone oil including chain polysiloxanes such as dimethyl polysiloxane, methylphenyl polysiloxane, and diphenyl polysiloxane; cyclic polysiloxanes such as octamethylcyclotetra siloxane, decamethylcyclopenta siloxane, dodecamthylcyclohexane siloxane; and modified siloxanes such as amino-modified polysiloxane, polyether-modified polysiloxane, alkyl-modified polysiloxane, and fluorine-modified polysiloxane; anionic surfactants such as fatty acid soaps (including sodium laurate and sodium palmitate), potassium laurylsulfate, and triethanolamine alkylsulfate; cationic surfactants such as trimethyl ammonium stearyl chloride, benzalconium chloride, and laurylamine oxide; amphoteric surfactants such as imidazoline-based amphoteric surfactants (including 2-cocoyl-2-imidazolinium hydroxide-1-carboxyethyloxy-2-sodium salt), betaine-based surfactants (including alkyl betaine, amide betaine, and sulfo betaine), and acylmethyl taurine; nonionic surfactants such as sorbitan fatty acid esters (including sorbitan monostearate and sorbitan sesquioleate), glycerin fatty acids (including glycerin monostearate), propyleneglycol fatty acid esters (including propyleneglycol monostearate), hydrogenated castor oil derivatives, alkyl glycerol, POE sorbitan fatty acid esters (including POE sorbitan monooleate and polyoxyethylene sorbitan monostearate), POE sorbitol fatty acid esters (including POE-sorbitol monolaurate), POE glycerol fatty acid esters (including POE-glyceryl monoisostearate), POE fatty acid esters (including polyethyleneglycol monooleate and POE distearate), POE alkyl ethers (including POE2-octyldodecyl ether), POE alkylphenyl ethers (including POE nonylphenylether), pluronic types, POE/POP alkyl ethers (including POE/POP2-decyltetradecyl ether), tetronic types, POE castor oil/hydrogenated castor oil derivatives (including POE castor oil and POE hydrogenated castor oil), sucrose fatty acid ester, and alkyl glycoside; polyols such as polyethylene glycol, glycerin, 1,3-butanediol, erythritol, sorbitol, xylitol, maltitol, propylene glycol, dipropylene glycol, diglycerin, isoprene glycol, 1,2-pentanediol, 2,4-hexanediol, 1,2-hexanediol, and 1,2-octanediol; moisture components such as sodium pyrrolidone crboxylate, lactate, and sodium lactate; fine particles such as mica, talc, kaolin, synthetic mica, calcium carbonate, magnesium carbonate, silicic anhydride (silica), aluminum oxide, and barium sulfate, whose surfaces may be treated; inorganic pigments such as red iron oxide, yellow iron oxide, black iron oxide, cobalt oxide, ultramarine blue, iron blue, titanium oxide, and zinc oxide, whose surfaces may be treated; pearl agents such as mica titanium, fish scale foil, and bismuth oxychloride, whose surfaces may be treated; organic dyes such as Red No. 202, Red No. 228, Red No. 226, Yellow No. 4, Blue No. 404, Yellow No. 5, Red No. 505, Red No. 230, Red No. 223, Orange No. 201, Red No. 213, Yellow No. 204, Yellow No. 203, Blue No. 1, Green No. 201, Purple No. 201, and Red No. 204, which may be laked; organic fine particles such as polyethylene powder, polymethyl methacrylate, nylon powder, and organopolysiloxane elastomer; p-aminobenzoate-based ultraviolet absorbents; anthranilate-based ultraviolet absorbents; salicylate-based ultraviolet absorbents; cinnamate-based ultraviolet absorbents; benzophenone-based ultraviolet absorbents; sugar-based ultraviolet absorbents; ultraviolet absorbents such as 2-(2'-hydroxy-5'-t-octylphenyl)benzotriazole, and 4-methoxy-4'-t-butyldibenzoylmethane; lower alcohols such as ethanol and isopropanol; vitamin A or derivatives thereof; vitamin B types such as vitamin B₆ hydrochloride, vitamin B₆ tripalmitate, vitamin B₆ dioctanoate, vitamin B₂ or derivatives thereof, vitamin B₁₂, and vitamin B₁₅ or derivatives thereof; vitamins including vitamin E types such as α-tocopherol, β-tocopherol, γ-tocopherol, and vitamin E acetate, vitamin D types, vitamin H, pantothenic acid, pantethine, and pyrroloquinoline quinone; and anti-fungus agents such as phenoxyethanol. A skin external preparation of the present invention can be produced by treating those components in accordance with a conventional method.

Hereinafter, the present invention will be described in more detail by examples, but it should not be limited to these examples.

### [Example 1]

A skin external preparation of the present invention was produced as a cream in accordance with the following formulation. First, components (A) and (B) were previously heated to 70°C, and the component (B) was added to the component (A) with stirring to produce a crude liposome. Then, the crude liposome was treated using an extruder, followed by granulation, to thereby yield a liposome-dispersed liquid. The liposome-dispersed liquid was added to a component (C) heated to 80°C, and the mixture was gradually added to a component (D), which had turned into a gel, with stirring to emulsify the mixture, followed by cooling with stirring, to thereby yield cream 1 in the form of a water-in-oil emulsion having droplets in which liposomes were comprised in the oil phase (in the form of a liposome-comprising water-in-oil emulsion). The same procedure was repeated except that a general dimethicone (uncrosslinked) was used instead of "Silicone KSG-16", to thereby produce cream 2 in the form of a liposome-comprising water-in-oil emulsion.

The same procedure was repeated except that hydrogenated lecithin was used instead of "Ceramide-TIC", to thereby produce Comparative Example 1 in the form of a liposome-comprising water-in-oil emulsion; except that bentonite that is an organically unmodified clay mineral was used instead of "Bentone 38V", to thereby produce Comparative Example 2 in the form of a liposome-comprising water-in-oil emulsion; except that hydrogenated lecithin was used instead of "Eldew PS-203", to thereby produce Comparative Example 3 in the form of a water-in-oil liposome-comprising emulsion; and except that hydrogenated lecithin was used instead of "Phytosterol", to thereby produce Comparative Example 4 in the form of a water-in-oil liposome-comprising emulsion.

**Table 1**

| Component (A) | % by mass |
|---|---|
| "Ceramide-TIC" | 0.5 |
| Phytosterol | 0.5 |
| (Phytosterol S Tama Biochemical Co., Ltd.; a mixture of β-sitosterol, stigmasterol, and campesterol) | |
| Hydrogenated lecithin | 1 |
| Macadamia nut oil | 1 |
| "Eldew PS-203" | 4 |
| (Ajinomoto Co., Inc.; di(octyldodecyl/phytosteryl) N-lauroylglutamate) | |
| 1,3-butanediol | 10 |
| Glycerine (B) | 10 |
| Water | 73 |
| (Subtotal) (C) | 100 |
| Squalane | 2 |
| Triglyceride isoocatanoate | 1 |
| Sucrose fatty acid ester | 0.2 |
| "Eldew PS-203" | 4 |
| Dimethicone | 27 |
| "Silicone KSG-16" | 7 |
| (Shin-Etsu Chemical Co., Ltd.; a cyclomethicone solution containing 25% of crosslinked dimethicone) | |
| Polyoxyethylene-modified dimethylpolysiloxane | 4 |
| "Bentone 38V" | 2.5. |
| Glycerine | 17 |
| 1,3-Butanediol (D) | 8 |
| "Liposome-dispersed liquid ((A)+(B))" | 20 |
| Water | 7.3 |
| Total | 100 |

### <Test Example 1>

For creams 1 and 2 and Comparative Examples 1 to 4, skin barrier function-improving effects were tested on the facies medialis brachii. Seven sites on the facies medialis brachii (2 cm x 4 cm), which had been stripped three times using an adhesive tape, were prepared. Then, one site was applied with 40 mg of cream 1 and wiped with an ethanol-impregnated cotton every 30 minutes; one site was applied with 40 mg of cream 2 and wiped with an ethanol-impregnated cotton every 30 minutes; one site was applied with 40 mg of the Comparative Example 1 and wiped with an ethanol-impregnated cotton every 30 minutes; one site was applied with 40 mg of the Comparative Example 2 and wiped with an ethanol-impregnated cotton every 30 minutes; one site was applied with 40 mg of the Comparative Example 3 and wiped with an ethanol-impregnated cotton every 30 minutes; one site was applied with 40 mg of the Comparative Example 4 and wiped with an ethanol-impregnated cotton every 30 minutes; and the other site was allowed to stand for 30 minutes without treatments and wiped with ethanol-impregnated cotton every 30 minutes. Ten minutes after wiping, the transepidermal water loss (TEWL) was determined using Tewameter (manufactured by Integral Corporation). The results are shown in Table 2. Creams 1 and 2 were found to deliver a ceramide to near the dermis that could not be wiped with an ethanol, resulting in improvement of the skin barrier function-improving effects.

**Table 2**

| Sample | TEWL |
|---|---|
| Cream 1 | 18.7 |
| Cream 2 | 17.8 |
| Comparative Example 1 | 25.6 |
| Comparative Example 2 | 23.1 |
| Comparative Example 3 | 25.4 |
| Comparative Example 4 | 24.4 |
| Control | 27.3 |

### <Test Example 2>

Subsequently, storage stability was tested for creams 1 and 2. Those creams were produced and allowed to stand at 5°C, 20°C, and 40°C, and the separated states were observed after one day and one month. Evaluation criteria are as follows.
AA: No change from a state immediately after production
A: No separation but slight reduction in viscosity
B: Partial separation and reduction in viscosity

The results are shown in Table 3. Cream 1 caused no separation even during low-temperature and high-temperature storage and was found to be extremely excellent in storage stability under severe conditions compared with cream 2. Therefore, a crosslinked dimethicone was found to contribute to improvement of the storage stability of a skin external preparation.

**Table 3**

| | | Cream 1 | Cream 2 |
|---|---|---|---|
| after one day | 5°C | AA | AA |
| | 20°C | AA | AA |
| | 40°C | AA | AA |
| after one month | 5°C | AA | A |
| | 20°C | AA | AA |
| | 40°C | AA | B |

### [Example 2]

The same test as Test Example 1 was performed for creams which were produced using different kinds of ceramides in cream 1 (creams 3 to 5). The evaluation results obtained by the procedure of Test Example 1 are shown in Table 5. The results revealed that the effects of delivering a ceramide were improved in the cases of all kinds of the ceramides.

**Table 4**

| Component (A) | % by mass |
|---|---|
| Ceramide described in Table 4 | 0.5 |
| Phytosterol (Phytosterol S Tama Biochemical Co., Ltd.; a mixture of β-sitosterol, stigmasterol, and campesterol) | 0.5 |
| Hydrogenated lecithin | 1 |
| Macadamia nut oil | 1 |
| "Eldew PS-203" (Ajinomoto Co., Inc.; | 4 |
| di(octyldodecyl/phytosteryl) N-lauroylglutamate) | |
| 1,3-Butanediol | 10 |
| Glycerine (B) | 10 |
| Water | 73 |
| (Subtotal) (C) | 100 |
| Squalane | 2 |
| Triglyceride isooctanoate | 1 |
| Sucrose fatty acid ester | 0.2 |
| "Eldew PS-203" | 4 |
| Dimethicone | 27 |
| "Silicone KSG-16" (Shin-Etsu Chemical Co., Ltd.; a cyclomethicone solution containing 25% of crosslinked dimethicone) | 7 |
| Polyoxyethylene-modified dimethylpolysiloxane | 4 |
| "Bentone 38V" | 2.5. |
| Glycerine | 17 |
| 1,3-Butanediol (D) | 8 |
| "Liposome-dispersed liquid" | 20 |
| Water | 7.3 |
| Total | 100 |

**Table 5**

| Sample | Ceramide | TEWL |
|---|---|---|
| Cream 3 | [Ceramide I] | 19.2 |
| Cream 4 | [Ceramide III] | 19.7 |
| Cream 5 | [Ceramide IIIA] | 21.1 |
| Control | | 28.7 |

### [Example 3]

The same test as Test Example 1 was performed for creams using different kinds of phospholipids in cream 1 (creams 6 to 8). The evaluation results obtained by the procedure of Test Example 1 are shown in Table 7. The results revealed that the effects of delivering a ceramide were improved in the cases of all kinds of the phospholipids.

**Table 6**

| Component (A) | % by mass |
|---|---|
| "Ceramide-TIC" | 0.5 |
| Phytosterol | 0.5 |
| (Phytosterol S Tama Biochemical Co., Ltd.; a mixture of β-sitosterol, stigmasterol, and campesterol) | |
| Phospholipid described in Table 6 | 1 |
| Macadamia nut oil | 1 |
| "Eldew PS-203" (Ajinomoto Co., Inc.; di(octyldodecyl/phytosteryl) N-lauroylglutamate) | 4 |
| 1,3-Butanediol | 10 |
| Glycerine (B) | 10 |
| Water | 73 |
| (Subtotal) (C) | 100 |
| Squalane | 2 |
| Triglyceride isooctanoate | 1 |
| Sucrose fatty acid ester | 0.2 |
| "Eldew PS-203" | 4 |
| Dimethicone | 27 |
| "Silicone KSG-16" | 7 |
| (Shin-Etsu Chemical Co., Ltd.; a cyclomethicone solution containing 25% of crosslinked dimethicone) | |
| Polyoxyethylene-modified dimethylpolysiloxane | 4 |
| "Bentone 38V" | 2.5 |
| Glycerine | 17 |
| 1,3-Butanediol (D) | 8 |
| "Liposome-dispersed liquid" | 20 |
| Water | 7.3 |
| Total | 100 |

**Table 7**

| Sample | Phospholipid | TEWL |
|---|---|---|
| Cream 6 | Soybean lecithin | 18.8 |
| Cream 7 | Egg-yolk lecithin | 19.1 |
| Cream 8 | Hydroxylated lecithin | 17.8 |
| Control | | 25.9 |

### [Example 4]

A test was performed on a dosage form comprising a ceramide in a normal emulsion form instead of the dosage form of cream 1 comprising a ceramide in a liposome form. First, components (A) and (B) were heated to 70°C, and the component (B) was added to the component (A) with stirring to produce a crude emulsion. Then, the crude emulsion was treated using a homomixer, followed by granulation, to thereby produce an oil-in-water emulsion. The emulsion was added to a component (C) heated to 80°C, and the mixture was gradually added to a component (D), which had turned into a gel, with stirring to emulsify the mixture, followed by cooling with stirring, to thereby yield cream 9 in the form of a water-in-oil emulsion (having droplets of an oil-in-water emulsion in the outer oil phase). The control is the same as that in Example 1. In the evaluation by the procedure of Test Example 1, the TEWL value of the control was 26.9, while the value of cream 9 was 19.8, which is a good result similar to that of cream 1. Then, it was found that a ceramide is more preferably comprised in the form of a liposome.

**Table 8**

| Component (A) | % by mass |
|---|---|
| "Ceramide-TIC" | 0.5 |
| Phytosterol (Phytosterol S Tama Biochemical Co., Ltd.; a mixture of β-sitosterol, stigmasterol, and campesterol) | 0.5 |
| Hydrogenated lecithin | 1 |
| Macadamia nut oil | 1 |
| Behenyl alcohol | 0.5 |
| "Eldew PS-203" (Ajinomoto Co., Inc.; di(octyldodecyl/phytosteryl) N-lauroylglutamate) | 4 |
| 1,3-Butanediol | 10 |
| Glycerine (B) | 10 |
| Water | 72.5 |
| (Subtotal) (C) | 100 |
| Squalane | 2 |
| Triglyceride isooctanoate | 1 |
| Sucrose fatty acid ester | 0.2 |
| "Eldew PS-203" | 4 |
| Dimethicone | 27 |
| "Silicone KSG-16" (Shin-Etsu Chemical Co., Ltd.; a cyclomethicone solution containing 25% of crosslinked dimethicone) | 7 |
| Polyoxyethylene-modified dimethylpolysiloxane | 4 |
| "Bentone 38V" | 2.5 |
| Glycerine | 17 |
| 1,3-Butanediol (D) | 8 |
| "Emulsion solution"((A)+(B)) | 20 |
| Water | 7.3 |
| Total | 100 |

### Industrial Applicability

The present invention can be applied to a skin external preparation such as a cosmetic.

## Claims

1. A skin external preparation in the form of a water-in-oil emulsion, comprising a ceramide, an organically modified clay mineral, a diester of N-acylglutamic acid, and a phytosterol.

2. The skin external preparation according to claim 1, further comprising a crosslinked dimethicone.

3. The skin external preparation according to claim 1 or 2, wherein the ceramide is comprised in the form of a liposome or a niosome.

4. The skin external preparation according to any one of claims 1 to 3, wherein the organically modified clay mineral is hectorite modified with a quaternary ammonium salt.

5. The skin external preparation according to any one of claims 1 to 4, wherein the diester of N-acylglutamic acid is di(phytostery/octyldodecyl) N-lauroylglutamate.

6. Non-therapeutic use of a composition in the form of a water-in-oil emulsion comprising a ceramide, an organically modified clay mineral, a diester of N-acylglutamic acid, and a phytosterol for protecting skin from external chemical irritation.

7. A composition in the form of water-in-oil emulsion comprising a ceramide, an organically modified clay mineral, a diester of N-acylglutamic acid and a phytosterol for ameliorating or preventing atopic dermatitis.

8. The skin external preparation according to any one of claims 3 to 5, wherein the liposome or niosome is dispersed in the inner aqueous phase.

9. The skin external preparation according to any one of claims 1 to 5, wherein the organically modified clay mineral is contained in the outer oil phase.

10. The skin external preparation according to any one of claims 1 to 5, wherein the diester of N-acyl glutamic acid is contained in the outer oil phase.

## Patentansprüche

1. Präparat zur äußerlichen Anwendung auf der Haut in Form einer Wasser-in-Öl-Emulsion, umfassend ein Ceramid, ein organisch-modifiziertes Tonmineral, einen N-Acylglutaminsäure-Diester und ein Phytosterol.

2. Präparat zur äußerlichen Anwendung auf der Haut nach Anspruch 1, das weiterhin ein vernetztes Dimethicon umfasst.

3. Präparat zur äußerlichen Anwendung auf der Haut nach Anspruch 1 oder 2, wobei das Ceramid in Form eines Liposoms oder eines Niosoms vorhanden ist.

4. Präparat zur äußerlichen Anwendung auf der Haut nach einem der Ansprüche 1 bis 3, wobei es sich bei dem organischmodifizierten Tonmineral um mit einem quaternären Ammoniumsalz modifiziertes Hektorit handelt.

5. Präparat zur äußerlichen Anwendung auf der Haut nach einem der Ansprüche 1 bis 4, wobei es sich bei dem N-Acylglutaminsäure-Diester um Di(phytostery/octyldodecyl)-N-lauroylglutamat handelt.

6. Nicht-therapeutische Verwendung einer Zusammensetzung in Form einer Wasser-in-Öl-Emulsion, umfassend ein Ceramid, ein organisch-modifiziertes Tonmineral, einen N-Acylglutaminsäure-Diester und ein Phytosterol, zum Schutz der Haut vor äußerer chemischer Reizung.

7. Zusammensetzung in Form einer Wasser-in-Öl-Emulsion, umfassend ein Ceramid, ein organisch-modifiziertes Tonmineral, einen N-Acylglutaminsäure-Diester und ein Phytosterol zur Linderung oder Prävention von atopischer Dermatitis.

8. Präparat zur äußerlichen Anwendung auf der Haut nach einem der Ansprüche 3 bis 5, wobei das Liposom oder Niosom in der inneren wässrigen Phase dispergiert ist.

9. Präparat zur äußerlichen Anwendung auf der Haut nach einem der Ansprüche 1 bis 5, wobei das organisch-modifizierte Tonmineral in der äußeren Ölphase enthalten ist.

10. Präparat zur äußerlichen Anwendung auf der Haut nach einem der Ansprüche 1 bis 5, wobei der N-Acylglutaminsäure-Diester in der äußeren Ölphase enthalten ist.

## Revendications

1. Préparation cutanée à usage externe sous la forme d'une émulsion eau dans l'huile renfermant un céramide, une argile minérale organiquement modifiée, un diester de l'acide N-acylglutamique et un phytostérol.

2. Préparation cutanée à usage externe selon la revendication 1, renfermant en outre une diméthicone réticulée.

3. Préparation cutanée à usage externe selon la revendication 1 ou la revendication 2, dans laquelle le céramide se présente sous la forme d'un liposome ou d'un niosome.

4. Préparation cutanée à usage externe selon l'une quelconque des revendications 1 à 3, selon laquelle l'argile minérale organiquement modifiée est de l'hectorite modifiée avec un sel d'ammonium quaternaire.

5. Préparation cutanée à usage externe selon l'une quelconque des revendications 1 à 4, dans laquelle le diester de l'acide N-acylglutamique est le di(phytostéry/octyldodecyl) N-lauroyl glutamate.

6. Utilisation non thérapeutique d'une composition sous la forme d'une émulsion eau dans l'huile renfermant un céramide, une argile minérale organiquement modifiée, un diester de l'acide N-acylglutamique et un phytostérol pour protéger la peau des irritations chimiques externes.

7. Composition sous la forme d'une émulsion eau dans l'huile renfermant un céramide, une argile minérale organiquement modifiée, un diester de l'acide N-acylglutamique et un phytostérol pour traiter ou prévenir la dermatite atopique.

8. Préparation cutanée à usage externe selon l'une quelconque des revendications 1 à 5, dans laquelle le liposome ou le niosome est dispersé dans la phase aqueuse discontinue.

9. Préparation cutanée à usage externe selon l'une quelconque des revendications 1 à 5, selon laquelle l'argile minérale organiquement modifiée est renfermée dans la phase huileuse continue.

10. Préparation cutanée à usage externe selon l'une quelconque des revendications 1 à 5, dans laquelle le diester de l'acide N-acylglutamique est renfermé dans la phase huileuse continue.
